# EUROPÄISCHE PATENTSCHRIFT

(11) **EP 0 380 146 B1**
(45) Veröffentlichungstag und Bekanntmachung des Hinweises auf die Patenterteilung: **18.08.1993**
(21) Anmeldenummer: 90105691.1
(22) Anmeldetag: 27.06.1986
(51) Int. Cl.: C07C 271/16, C07C 269/04

(54) **Verfahren zur Herstellung von N-omega-Hydroxyalkyl-, N-omega-Hydroxy-oxa-alkyl- und N-omega-Hydroxy-polyoxa-alkylcarbaminsäureestern**
Process for the preparation of N-omega-hydroxyalkyl, N-omega-hydroxy-oxa-alkyl and N-omega-hydroxy-polyoxa-alkyl carbamates
Procédé pour la préparation de N-oméga-hydroxyalkyl-N-oméga-hydroxy-oxa-alkyl et N-oméga-hydroxy-polyoxa-alkyl carbamates

(30) Priorität: 03.07.1985 DE 3523692
(43) Veröffentlichungstag der Anmeldung: 01.08.1990
(62) Teilanmeldung aus: 86108777.3
(73) Patentinhaber: BASF Aktiengesellschaft, 67063 Ludwigshafen (DE)
(72) Erfinder: Merger, Franz, Dr., D-6710 Frankenthal (DE); Schwarz, Wolfgang, Dr., D-7507 Pfinztal (DE)

(56) Entgegenhaltungen:
- EP-A- 0 027 940
- US-A- 1 927 858
- US-A- 2 718 516

## Beschreibung

Die vorliegende Erfindung betrifft ein neues Verfahren zur Herstellung von N-ω-Hydroxialkyl-, N-ω-Hydroxi-oxa-alkyl- und N-ω-Hydroxi-polyoxa-alkyl-carbaminsäureestern der allgemeinen Formel I

HO-A-NHCO₂R' (I),

in der A für einen Alkylenrest, einen Oxa-alkylenrest oder einen Polyoxa-alkylenrest mit jeweils 2 bis 12 C-Atomen und R' für einen gegebenenfalls ein Sauerstoffatom enthaltenden Alkylrest mit 1 bis 6 C-Atomen stehen.

Die Verbindungen I sind als Zwischenprodukte zur Herstellung von ω-Isocyanato-alkyl-(meth)-acrylaten der allgemeinen Formel II 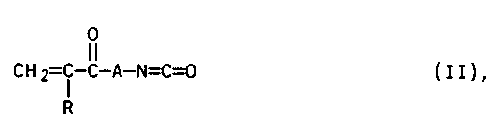
in der R für H- oder CH₃- steht, von Bedeutung. Die (Meth)acrylate II können als Ausgangsprodukte zur Herstellung von Pharmazeutika und Insektiziden sowie als Monomere bzw. Comonomere für die Herstellung von Polymerisaten und Copolymerisaten eingesetzt werden.

Bisher wurden die Verbindungen I meist durch Umsetzen von Aminoalkanolen mit Chlorkohlensäureestern gewonnen. So offenbart z.B. die US-PS 2 485 855 die Herstellung des 2-Hydroxiethylcarbaminsäureethylesters aus 2-Aminoethanol und Chlorkohlensäureethylester. Nach Iwakura (Chem. High Polymers Japan 2 (1945), 305) erhält man beim Erwärmen von 6-Hydroxihexanoylazid mit Ethanol in Benzol N-(5-Hydroxipentyl)carbaminsäureethylester. Die US-PS 1 927 858 schließlich lehrt die Bildung von N-Hydroxialkylcarbaminsäureestern aus Aminoalkoholen und Dialkylcarbonaten. Nachteilig an allen vorgenannten Verfahren ist, daß sie verhältnismäßig toxische und teure Edukte verwenden.

Aufgabe der vorliegenden Erfindung war es, diesem Nachteil abzuhelfen. Demgemäß wurde ein neues Verfahren zur Herstellung von Verbindungen I gefunden, das dadurch gekennzeichnet ist, daß man einen Aminoalkohol der allgemeinen Formel III

HO-A-NH₂ (III),

mit Harnstoff und einem Alkohol der allgemeinen Formel IV

R'OH (IV),

zu einer Verbindung I umsetzt.

Es ist überraschend, daß die Verbindungen I nach dem erfindungsgemäßen Verfahren in guter Ausbeute erhalten werden, zumal im Hinblick auf die Bifunktionalität der Aminoalkoholkomponente eine Vielzahl verschiedener Reaktionsprodukte denkbar ist. Selbst bei Verwendung von Aminoethanol und Aminopropanol, wo durch Ringschluß die ausschließliche Bildung von 2-Oxo-tetrahydro-1,3-oxazolidin bzw. 2-Oxo-tetrahydro-1,3-oxazin zu erwarten war, wurden die gewünschten Carbaminsäureester, nämlich der N-(2-Hydroxiethyl)-bzw. N-(3-Hydroxipropyl)-carbaminsäureester in befriedigenden Ausbeuten erhalten.

Als Aminoalkohole werden für das neue Verfahren solche vorgezogen, deren Alkylenreste 4 bis 12, insbesondere 4 bis 8 C-Atome enthalten. Beispiele für geeignete Aminoalkohole sind 2-Aminoethanol, 3-Aminopropanol, 4-Aminobutanol, 5-Aminopentanol, 6-Aminohexanol, 3-Amino-isobutanol, 2-Aminobutanol-1, 3-Methyl-5-aminopentanol und 2,2-Dimethyl-3-aminopropanol-1. Geeignete Oxa-aminoalkanole sind z.B. 3-Oxa-5-aminopentanol-1, 3-Oxa-6-aminohexanol-1, 2,2-Dimethyl-4-oxo-7-aminoheptanol und 5-Oxa-8-aminooctanol-1. Als Polyoxaaminoalkohol sei beispielhaft 3,6-Dioxa-9-aminononanol-1 genannt. Als Alkohole werden vorzugsweise 1 bis 6 C-Atome enthaltende Alkanole, wie Methanol, Ethanol, Propanol, n-Butanol, Isobutanol und Hexanol eingesetzt. Von besonderem Interesse sind Propanol und Butanol. Ferner geeignet als Alkoholkomponente sind Cycloalkanole wie besonders Cyclohexanol und araliphatische Alkohole, wie Benzylalkohol und 2-Phenylethanol sowie ferner Etheralkohole wie Methoxiethanol. Bei der Umsetzung wendet man im allgemeinen ein Molverhältnis von Aminoalkohol zu Harnstoff zu Alkohol von 1:0,7 bis 5:1 bis 100, vorzugsweise von 1:1 bis 1,5:5 bis 50 an. Dies gilt auch beim Einsatz von Amino-oxa-alkoholen und Amino-polyoxa-alkoholen der genannten Art. Sei der praktischen Durchführung des Verfahrens können die Ausgangskomponenten in dem angegebenen Verhältnis vermischt und meist bei Temperaturen von 170 bis 250°C, insbesondere bei 180 bis 235°C umgesetzt werden. Dabei kann man bei Drücken von 0,1 bis 50 bar arbeiten, entsprechend dem Dampfdruck des verwendeten Alkohols, doch wird vorteilhaft ein Reaktionsdruck eingestellt derart, daß die Reaktionsmischung siedet, damit der bei der Reaktion entstehende Ammoniak möglichst vollständig abgetrennt wird. Das Abtrennen von Ammoniak während der Reaktion kann auch z.B. durch Hindurchleiten von indifferenten Gasen unterstützt werden. Anstelle von Harnstoffen kann bei der Reaktion auch Biuret, Triuret usw. gegebenenfalls zusammen mit Harnstoff eingesetzt werden. Anstelle von Harnstoff und Alkohol kann man auch die entsprechenden Carbaminsäureester, gegebenenfalls unter Zusatz von Alkohol und/oder Harnstoff einsetzen.

Bei dem Verfahren kann man die Umsetzung auch unter Zusatz geringer Mengen Katalysatoren durchführen. Als solche kommen besonders Chloride, Bromide, Sulfate, Phosphate, Nitrate, Borate, Alkoholate, Phenolate, Sulfonate, Oxide, Oxidhydrate, Hydroxide, Carboxilate, Chelatverbindungen, Carbonate, Thiocarbamate und Dithiocarbamate vorzugsweise des Lithiums, Calciums, Aluminiums, Zinns, Wismuts, Antimons, Kupfers, Zinks, Titans, Vanadins, Chroms, Molybdäns, Mangans, Eisens, Nickels und Kobalts in Frage. Von besonderem Interesse sind Verbindungen von Eisen, Kobalt, Nickel, Zink, Zinn und Titan.

Derartige Katalysatoren können im allgemeinen in Mengen von 0,0001 bis 0,1, vorzugsweise von 0,0005 bis 0,05 äquivalent Metallion, bezogen auf den Aminoalkohol eingesetzt werden.

Die nach dem neuen Verfahren gebildeten gewünschten N-ω-Hydroxialkyl-, N-ω-Hydroxi-oxa-alkyl- oder N-ω-Hydroxi-polyoxa-alkyl-carbaminsäureester können nach Abdestillieren von überschüssigem Alkohol und eventuellen Nebeprodukten durch Destillation im Vakuum oder durch Kristallisation gereinigt wreden.

Die so erhältlichen N-ω-Hydroxialkyl-, N-ω-Hydroxi-oxa-alkyl- oder N-ω-Hydroxi-polyoxa-alkyl-carbaminsäureester können durch Umsetzen mit (Meth)acrylsäurealkylestern oder (Meth)acrylsäureanhydrid verestert und die dabei erhaltenen ω-Alkoxicarbamoyl-alkyl-, -oxa-alkyl- und -polyoxa-alkyl(meth)acrylate durch Erhitzen in Verbindungen II und Alkohol gespalten werden.

### Beispiele

### Beispiel 1

In einem 1 l-Rührautoklaven mit aufgesetzter Druckkolonne und Druckregelventil werden 35,6 g 4-Aminobutanol, 26,4 g Harnstoff, 592 g n-Butanol und 68 mg Zinn(II)chlorid 5 Stunden bei 5 bar unter Rückfluß und Abnahme von Ammoniak gekocht. Man erhielt 636 g einer gelblichen Flüssigkeit. Eine Analyse des Reaktionsgemisches (Gelpermeationschromatographie, Methode des externen Standards) zeigte, daß die Umwandlung in N-(4-Hydroxibutyl)-carbaminsäurebutylester 84 % betrug.

Nach Abdestillieren von überschüssigem Butanol und Carbaminsäurebutylester wurde der Rückstand über einen Dünnschichtverdampfer destilliert. Das Produkt geht bei 140°C/0,1 mbar über und erstarrt in der Vorlage kristallin. Es wurden 59,7 g (79 %) N-(4-Hydroxibutyl)carbaminsäureester (Reinheit > 99 %) erhalten. Eine Probe wurde aus Essigsäureethylester umkristallisiert und schmilzt bei 51°C.

### Beispiel 2

In einem 1 l-Rührautoklaven mit aufgesetzter Druckkolonne und Druckregelventil werden 42 g 5-Amino-3-oxapentanol, 26,4 g Harnstoff, 592 g n-Butanol 2 Stunden bei 230°C und 16 bar unter Abnahme von Ammoniak am Rückfluß gekocht. Man erhielt 628 g einer gelblichen Flüssigkeit, deren Analyse mittels Gelpermeationschromatographie eine Umwandlung von 92 % anzeigte. Nach Abdestillieren von überschüssigem Butanol und Carbamat wurde der Rückstand von vier Ansätzen destilliert. Das Produkt geht bei 132°C/0,1 mbar über. Es wurden 286 g (87 %) N-(5-Hydroxi-3-oxapentyl)carbaminsäurebutylester erhalten.

## Patentansprüche

1. Verfahren zur Herstellung von Verbindungen der allgemeinen Formel I
HO-A-NHCO₂R' (I),
in der A für einen Alkylenrest, einen Oxa-alkylenrest oder einen Polyoxa-alkylenrest mit jeweils 2 bis 12 C-Atomen und R' für einen gegebenenfalls ein Sauerstoffatom enthaltenden Alkylrest mit 1 bis 6 C-Atomen stehen, dadurch gekennzeichnet, daß man einen Aminoalkohol der allgemeinen Formel III
HO-A-NH₂ (III),
mit Harnstoff und einem Alkohol der allgemeinen Formel IV
R'OH (IV),
bei 170 bis 250°C unter Abtrennung von Ammoniak umsetzt.

2. Verfahren nach Anspruch 1, dadurch gekennzeichnet, daß man die Umsetzung bei 180 bis 235°C ausführt.

3. Verfahren nach Anspruch 1 oder 2, dadurch gekennzeichnet, daß das Molverhältnis Aminoalkohol III:Harnstoff:Alkohol IV 1:1 bis 1,5:5 bis 50 beträgt.

## Claims

1. A process for preparing compounds of the formula I
HO-A-NHCO₂R' (I)
where A is alkylene, oxa-alkylene or polyoxa-alkylene with, in each case, 2-12 carbon atoms and R' is alkyl which has 1-6 carbon atoms and may contain an oxygen atom, which comprises reacting an amino alcohol of the formula III
HO-A-NH₂ (III)
with urea and an alcohol of the formula IV
R'OH (IV)
at from 170 to 250°C with removal of ammonia.

2. A process as claimed in claim 1, wherein the reaction is carried out at from 180 to 235°C.

3. A process as claimed in claim 1 or 2, wherein the amino alcohol III:urea:alcohol IV molar ratio is 1:1-1.5:5-50.

## Revendications

1. Procédé de préparation de composés de formule générale I
HO-A-NHCO₂R' (I)
dans laquelle A est mis pour un reste alkylène, un reste oxa-alkylène ou un reste polyoxa-alkylène à 2-12 atomes de carbone chacun, et R' est mis pour un reste alkyle à 1-6 atomes de carbone contenant éventuellement un atome d'oxygène, caracterisé en ce qu'on fait réagir un aminoalcool de formule générale III
HO-A-NH₂ (III)
avec de l'urée et un alcool de formule générale IV
R'OH (IV)
à une température de 170 à 250°C, avec séparation d'ammoniac.

2. Procédé selon la revendication 1, caractérisé en ce qu'on conduit la réaction à une température de 180 à 235°C

3. Procédé selon la revendication 1 ou 2, caractérisé en ce que le rapport molaire aminoalcool III : urée : alcool IV s'élève à 1 : 1-1,5 : 5-50.
